## Europäisches Patentamt

## European Patent Office

## Office européen des brevets

(19)

(11) **EP 1 469 102 A1**

(12) ## EUROPEAN PATENT APPLICATION

(43) Date of publication:
**20.10.2004 Bulletin 2004/43**

(51) Int Cl.⁷: **C25B 1/28**, A61L 2/03

(21) Application number: **04007700.0**

(22) Date of filing: **30.03.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL LT LV MK** | • **Saha, Madhu Sudan**<br>**Fujisawa-shi Kanagawa 252-0816 (JP)**<br>• **Uno, Masaharu**<br>**Fujisawa-shi Kanagawa 252-0816 (JP)**<br>• **Nishiki, Yoshinori**<br>**Fujisawa-shi Kanagawa 252-0816 (JP)**<br>• **Furuta, Tsuneto**<br>**Fujisawa-shi Kanagawa 252-0816 (JP)** |
| (30) Priority: **31.03.2003 JP 2003095501** | |
| (71) Applicant: **PERMELEC ELECTRODE LTD.**<br>**Fujisawa-shi, Kanagawa 252-0816 (JP)** | (74) Representative: **Grünecker, Kinkeldey,**<br>**Stockmair & Schwanhäusser Anwaltssozietät**<br>**Maximilianstrasse 58** |
| (72) Inventors:<br>• **Ohsaka, Takeo**<br>**Machida-shi Tokyo 194-0012 (JP)** | **80538 München (DE)** |

(54) **Method for the electrolytic synthesis of peracetic acid and sterilizing-cleaning method and apparatus**

(57) Simple and economic electrolytic synthesis of peracetic acid is performed, and cold drink containers, etc., are sterilized/cleaned with an aqueous solution of peracetic acid thus obtained. Peracetic acid is electrolytically synthesized from acetic acid and/or acetate and an oxygen-containing gas as starting materials in the presence of a solid acid catalyst in an electrolytic cell. An object to be cleaned is sterilized/cleaned with an aqueous solution of peracetic acid thus obtained. Peracetic acid can be simply obtained at a reduced cost. The sterilizing/cleaning of an object to be cleaned with peracetic acid can be conducted efficiently.

FIG. 1

**EP 1 469 102 A1**

**Description**

**FIELD OF THE INVENTION**

The present invention relates to a method for the electrolytic synthesis of peracetic acid and a method and apparatus for sterilizing an object to be cleaned using the peracetic acid thus synthesized.

**DESCRIPTION OF THE RELATED ART**

Unlike sterilizing/cleaning with effective chlorine, disinfection of containers for cold drinks, beer, etc. and medical tools such and endoscope with water containing peroxide such as hydrogen peroxide and peracetic acid involves no generation of trihalomethane, etc. and leaves low toxicity residues if any. Thus, this method is widely used as a method excellent in safety and environmental adaptability. In general, concentrated peracetic acid solution is carried into plants where it is then diluted for use in sterilizing/cleaning.

Peracetic acid is a disinfectant effective for all microorganism including germ. A 0.2% aqueous solution of peracetic acid can kill germ in a shorter period of time than glutaral. Peracetic acid undergoes little deactivation even in the presence of organic materials, exerts an effect on germ even at low temperatures and exhibits a higher sterilizing power at lower pH value. A solution containing 2,000 ppm or more of peracetic acid is capable of killing germ when brought into contact with the object for 5 minutes at ordinary temperature.

Hydrogen peroxide, too, has a sterilizing effect and anti-organism properties almost comparable to that of glutaral. In Europe and America, a stabilized hydrogen peroxide having a concentration of 6% or more is utilized for disinfection of medical tools such as endoscope.

In order to discharge hydrogen peroxide-containing water used for sterilizing as waste water, it is necessary that TOC value be reduced in a biological disposal tank. In other words, when hydrogen peroxide remains, it is likely that the oxidizing power of hydrogen peroxide can kill active sludge. In order to avoid this problem, the decomposition of hydrogen peroxide is needed. To this end, the use of various reducing agents is indispensable, adding to cost.

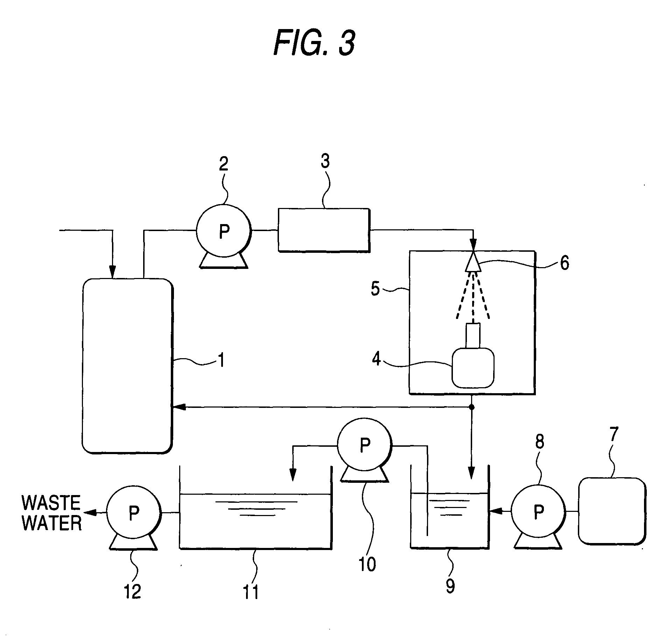

An example of the related art method for sterilizing/cleaning an object to be cleaned with a peroxide will be described hereinafter in connection with Fig. 3.

In the related art method, a peroxide such as peracetic acid and hydrogen peroxide is supplied into a chemical storage tank 1 where it is stored in the form of aqueous solution. Subsequently, using a first circulating pump 2, the aqueous solution in the chemical storage tank 1 is passed through a heating device 3 where it is then heated. The aqueous solution is supplied into a sterilizing/cleaning chamber 5 receiving an object 4 to be cleaned where it is then sprayed onto the object 4 through a nozzle 6.

The aqueous solution of peroxide used for sterilizing/cleaning is then partially returned to the chemical storage tank 1 for reuse.

The sterilizing/cleaning chamber 5 is also connected to a reduction tank 7 which is connected to a reduction tank 7 through a feed pump 8. The rest of the aqueous solution of peroxide which has not been returned to the chemical storage tank 1 is introduced into the reduction tank 7. In the reduction tank 7, peracetic acid or hydrogen peroxide in the aqueous solution of peroxide is decomposed with a reducing agent. The aqueous solution thus decomposed is passed to a biological disposal tank 11 by a second circulating pump 10 where it is then treated to produce a clean aqueous solution having a reduced TOC value which is then discharged to rivers or the like through a discharge pump 12.

In the related art method for sterilizing/cleaning of an object to be cleaned, concentration solution of peracetic acid (about 6%) is carried to plants where it is then diluted (up to 20-3,500 ppm) for circulation (chemical storage tank 1 → heating device 3 → sterilizing/cleaning chamber 5 → chemical storage tank 1 in Fig. 3). The aqueous solution of peroxide is then discarded after a predetermined period of use.

In order to discard the aqueous solution of peroxide, the aqueous solution of peroxide in the sterilizing/cleaning chamber 5 is introduced into the reduction tank 9 where peroxide remaining in the aqueous solution of peroxide is then neutralized with a reducing agent. Thereafter, the aqueous solution of peroxide is passed to the biological disposal tank where it is treated to produce an aqueous solution having a reduced TOC value which is then discharged.

The practice of this method requires a vast site and a huge disposal cost. Further, since concentrated peracetic acid solution which is a starting material needs to be carried externally, it takes much labor to keep or store the concentrated peracetic acid solution. Thus, this method is not a safe method. Moreover, this method is disadvantageous in that the addition of a stabilizer for preventing the spontaneous decomposition of peracetic acid is required.

A medical sterilizing method involving the use of an aqueous solution of a composition made of peracetic acid and hydrogen peroxide is disclosed in JP-A-2001-70412. However, a method for the synthesis of sterilizer on site is not disclosed. JP-A-2002-307081 discloses that activated charcoal can be used for the decomposition of peroxide, organic materials can be recovered through an ion-exchange membrane and NF (nanofilter), RO (reverse osmosis membrane), MF (membrane filter) or the like can be used to separate the dead body of living things, making it possible to recover and reuse the used sterilizing water which has heretofore been wasted. However, acetic acid thus recovered can no longer be used but discarded. Thus, basic problems have been left unsolved.

Electrolytic acidic water is one of recent topics about on-site production of sterilizing water. This electrolytic acidic water was approved as a disinfectant having a sterilizing capacity by the Ministry of Health, Labour and Welfare (Notification No. 212 of the Ministry of Health, Labour and Welfare dated June 10, 2002) and has been widely used more and more as hygienically-controlled water in hospitals and food plants. It is reported that the electrolytic acidic water has a sterilizing effect on legionella also. However, the electrolytic acidic water is mainly a hypochlorous acid-based sterilizer and thus causes problems when used commonly and leaves something to be desired in safety.

Peracetic acid, which is excellent as a sterilizer, is synthesized by oxidizing acetaldehyde by oxygen activated at a temperature of about 100-200°C on an industrial basis. However, this procedure requires a high temperature operation that can be difficultly conducted on site.

It is also known that when hydrogen peroxide is reacted with acetic acid or acetic anhydride in the presence of an acid catalyst, peracetic acid is synthesized. However, this method requires the use of sulfuric acid, as an acid catalyst, which can be difficultly separated from the reaction product, giving an apprehension that the safety can be impaired when conducted on site.

This method has been improved. As a result, a proper solid acid catalyst has been found from the standpoint of separatability, safety, reaction efficiency, etc. This solid catalyst can be easily separated from the reaction product and has an enhanced safety. Although this method allows on-site synthesis of peracetic acid, it requires the storage of hydrogen peroxide and thus cannot provide a simple sterilizing system.

For the purpose of eliminating these disadvantages, a method for electrolytic synthesis of peracetic acid is disclosed in JP-T-2003-506120. In accordance with this method, oxygen gas is electrolyzed to obtain peroxide species (e.g., peroxide ions, peroxide radicals, hydrogen peroxide) which are then reacted with acetylsalicylic acid which is a peracetic acid precursor or the like to obtain peracetic acid. However, since this method involves the use of acetylsalicylic acid or the like, which is expensive, as a peracetic acid precursor and the electrolytic synthesis of peroxide species which are then reacted with the peroxide precursor to synthesize peracetic acid, it requires a prolonged step. Thus, this method cannot be regarded as an economic synthesis method.

## SUMMARY OF THE INVENTION

Accordingly, one object of the present invention is to provide a simpler method for the electrolytic synthesis of an aqueous solution containing peracetic acid having a high sterilizing/cleaning capacity, particularly on site.

Another object of the present invention is to provide a method and apparatus for efficient sterilizing/cleaning with peracetic acid thus electrolytically synthesized.

A first embodiment of the present invention concerns a method for the sterilizing/cleaning of an object with an aqueous solution of a peroxide, which comprises sterilizing/cleaning the object with an aqueous solution containing a peracetic acid electrolytically synthesized from acetic acid and/or acetate and an oxygen-containing gas as starting materials.

A second embodiment of the present invention concerns a sterilizing/cleaning apparatus using a peroxide comprising an electrolytic cell for performing electrolysis while being supplied with acetic acid and/or acetate and an oxygen-containing gas to synthesize an aqueous solution containing peracetic acid and hydrogen peroxide at the cathode, a sterilizing/cleaning chamber for allowing the aqueous solution produced in the electrolytic cell to come in contact with an object, a filter disposed downstream the chamber for filtering the aqueous solution of a peroxide containing peracetic acid and hydrogen peroxide from the sterilizing/cleaning chamber to remove the dead body of living things and a unit for circulating the aqueous solution of peroxide filtered through the filter to the electrolytic cell.

A third embodiment of the present invention further concerns a method for the electrolytic synthesis of peracetic acid which comprises electrolytically synthesizing peracetic acid from acetic acid and/or acetate and an oxygen-containing gas as starting materials in the presence of a solid acid catalyst.

## BRIEF DESCRIPTION OF THE DRAWINGS

Fig. 1 is a schematic diagram illustrating an embodiment of the sterilizing/cleaning method with peroxide according to the present invention;
Fig. 2 is a schematic longitudinal sectional view of the electrolytic cell of Fig. 1; and
Fig. 3 is a schematic diagram illustrating an embodiment of the related art sterilizing/cleaning method with peroxide.

In the drawings:

| | |
|---|---|
| 21 | Electrolytic cell |
| 22 | Oxygen supplying device |
| 23 | Storage tank |
| 25 | Concentration sensor |
| 26 | Object to be cleaned |
| 27 | Cleaning chamber |
| 28 | Nozzle |
| 30 | Filter for removing decomposition product |
| 41 | Anode |
| 42 | Oxygen gas cathode |
| 43 | Membrane |
| 44 | Particulate solid acid catalyst |

## DETAILED DESCRIPTION OF THE INVENTION

The present invention will be further described hereinafter.

The inventors confirmed that peracetic acid can be electrolytically synthesized from inexpensive materials in one stage unlike the aforementioned related art technique involving the two stage electrolytic synthesis using expensive materials. It was further found that the sterilizing/cleaning of peracetic acid thus obtained optionally with hydrogen peroxide makes it possible to make sterilizing/cleaning of the object to be cleaned at a high efficiency.

In other words, although it was known that hydrogen peroxide can be electrolytically synthesized efficiently by the electrolytic reduction of oxygen, it has been found that the electrolysis of acetic acid, too, makes it possible to synthesize hydrogen peroxide. Then, a mixture of acetic acid hydrogen peroxide electrolytically synthesized was brought into contact with a related art solid acid catalyst. As a result, it was confirmed that peracetic acid can be synthesized.

It was then studied on the basis of this knowledge to see if the electrolytic synthesis of peracetic acid itself, which has not heretofore been reported, is made possible. If this electrolytic synthesis is made possible, it means that peracetic acid can be synthesized efficiently while controlling the concentration of the reaction solution.

Electrolysis was conducted on acetic acid as a starting material in an electrolytic cell filled with a solid acid while an oxygen-containing gas is being supplied thereinto. As a result, it was confirmed that peracetic acid and hydrogen peroxide can be synthesized at the same time.

The oxygen reduction reaction in an electrolytic cell usable in the invention involves a reaction of active species which can be present only on or in the vicinity of the active surface of electrode, an indirect electrolytic reaction and an indirect reaction with relatively stable chemical species such as hydrogen peroxide to synthesize peracetic acid.

The main reaction on the anode and cathode are represented by the following equations (1) and (2). As the cathode there is preferably used a gas diffusion electrode because it allows easy progress of reduction of oxygen. It is presumed that acetic acid reacts with $O_2^-$ produced by electrolytic reduction or an active oxygen adsorption specie $O^*$ produced on the surface of the cathode to cause the progress of direct oxidation reaction on the electrode as shown in the equation (3). As shown in the equation (4), it is also presumed that acetic acid reacts partially with hydrogen peroxide to synthesize peracetic acid.

In the presence of a solid acid catalyst, the reaction of the equation (4) proceeds in equilibrium. The donation of proton causes the bond of oxygen atoms in hydrogen peroxide to be severed. One of the OH radicals then reacts with proton to produce water while the other reacts with acetic acid to produce peracetic acid.

Further, in the case where the catholyte and the anolyte are separated from each other, hydrogen peroxide generated by the cathode can be easily decomposed on the anode as shown in the equation (5) because it is active. The resulting electrolyte contains acetic acid or acetate and peracetic acid as starting materials, making it possible to obtain an aqueous solution containing hydrogen peroxide only in a slight amount.

$$\text{Anode: } 2H_2O = O_2 + 4H^+ + 4e(1.23 \text{ V}) \tag{1}$$

$$\text{Cathode: } O_2 + 2H^+ + 2e = H_2O_2 \ (0.683 \text{ V}) \tag{2}$$

$$Cathode: CH_3COOH + O^* = CH_3COOOH \tag{3}$$

$$H_2O_2 + CH_3COOH + (H^+) = CH_3COOH(OH\text{-}) + H_2O =$$

$$H_2O + CH_3COOOH + (H^+) \tag{4}$$

$$H_2O_2 = O_2 + 2H^+ + e \ (0.68 \ V) \tag{5}$$

The catalyst employable herein is an acid catalyst. A solid protonic acid can be considered to have an excellent utility from the standpoint of separatability. Examples of the protonic acid include sulfone resins. As the sulfone resins there can be used resin polymers commercially available with various trade names, e.g., AMBERLYST and DOWEX in the form of bead or granule. These resins are formed by a polystyrene-divinylbenzene skeleton having a sulfone group as a functional group. However, a styrene skeleton is disadvantageous in that it can be easily decomposed by peracetic acid thus produced or hydrogen peroxide and thus cannot be used over an extended period of time, though being inexpensive.

As a resin having an excellent chemical resistance which can substitute for the aforementioned resin polymer there may be used a fluororesin having a sulfone group as an ion-exchange group such as Nafion, which is a commercially available product. Nafion is produced as a copolymer of tetrafluoroethylene and perfluoro[2-(fluorosulfonylethoxy)-propyl]vinylether.

This resin is preferably in the form of powder or grain having a diameter of from 0.01 mm to 3 mm. Nafion is particularly favorable from the standpoint of activity and chemical stability as solid acid. It is presumably because a fluorinated carbon resin which is a skeleton of Nafion is chemically inert to active oxygen or peracetic acid thus produced and thus can be little decomposed.

On the other hand, an organic-inorganic composite catalyst, too, can be used to prepare a Nafion-silica composite for example.

As a catalyst material having an ion exchange capability usable as a solid catalyst there may be used a hydrocarbon-based resin such as styrene-based resin, acrylic resin and aromatic polymer-based resin besides the aforementioned commercially available particulate ion-exchange resins. From the standpoint of corrosion resistance, fluorinated resin-based catalyst materials are desirable. Further, an ion-exchanging component can be formed in a proper porous support material.

The porosity of the material is preferably 20-90% taking into account the uniform dispersion and resistivity of the solution. The size of the particulate material is preferably 0.1-10 mm. The more the added amount of the solid acid is, the higher are the concentration of the product thus obtained and the rate at which the product can be obtained. In actuality, however, the amount of the solid acid to be added is limited from the standpoint of device cost. In practice, the volume of the catalyst is preferably from half to one tenth of that of the starting material solution.

As an oxygen gas cathode catalyst suitable for the acceleration of the reaction of active oxygen with acetic acid and the production of hydrogen peroxide there may be used a platinum group metal, oxide thereof, carbon-based material such as graphite and electrically-conductive diamond or polyaniline or thiol (SH group-containing organic material). These catalysts may be used as they are in tabular form or formed on a corrosion-resistant sheet made of stainless steel, zirconium, silver, carbon or the like, a metal gauze, a sintered powder or a sintered metal fiber to a thickness of 1-1,000 $g/m^2$ by a thermal decomposition method, a resin fixing method, a composite plating method or the like.

As the cathode electricity supplier there may be used carbon, a metal such as nickel and titanium or alloy or oxide thereof. In order to facilitate the supply and removal of the reaction product gas or solution, a hydrophobic or hydrophilic material is preferably supported on the cathode electricity supplier in dispersion. The formation of a hydrophobic sheet on the side of the cathode opposite the anode makes it possible to control the supply of gas onto the reaction surface to advantage.

The amount of oxygen to be supplied onto the cathode is preferably about 1.1-10 times the calculated value. As the oxygen-containing gas to be used as a starting material there may be used air, oxygen-rich air obtained by subjecting nitrogen by separation and concentration, oxygen gas supplied from a commercially available bomb or the like. The oxygen-containing gas is normally supplied into a gas chamber, if provided on the back side of the electrode, but may be bubbled in the electrolyte to be supplied into the electrolytic cell.

Examples of the anode catalyst include lead oxide, tin oxide, platinum, DSA, iron, graphite, and electrically-conductive diamond. These catalysts may be used as they are in tabular form or formed on a corrosion-resistant sheet made of titanium, niobium and tantalum or the like, a metal gauze, a sintered powder or a sintered metal fiber to a thickness of 1-500 $g/m^2$ by a thermal decomposition method, a resin fixing method, a composite plating method, CVD or the like.

The material which can be used as an electrode substrate needs to be so corrosion-resistant as to prolong its life and cause no contamination of the treated surface. As the anode electricity supplier there is preferably used a valve metal or alloy thereof.

Anode reaction normally involves the generation of oxygen. In the invention, however, the kind of the anode used or the electrolysis conditions are adjusted to increase the produced amount of peracetic acid. Further, by properly selecting the anode catalyst, an oxidation reaction involving the decomposition of organic materials in the aqueous solution of acetic acid recovered into inorganic materials (e.g., carbon dioxide, carbonic acid ion), too, proceeds, making it possible to contribute the maintenance of the quality of circulating water.

The partition of the electrolytic cell into an anode chamber and a cathode chamber by a membrane makes it possible to prevent various ions produced on the anode and cathode from being consumed by the counter electrode and hence stably maintain the active materials produced by the electrolytic reaction. At the same time, this arrangement facilitates the progress of electrolysis even when the electrical conductivity of the solution is low. Examples of membranes employable herein include neutral membrane and ion-exchange membrane. The ion-exchange membrane to be used herein may be either fluororesin-based or hydrocarbon resin-based. The former is desirable from the standpoint of corrosion resistance.

On the other hand, electrolysis may be conducted without any membrane. By selectively oxidation- decomposing hydrogen peroxide produced on the anode, the ratio of concentration of peracetic acid and hydrogen peroxide can be adjusted.

Referring to electrolysis conditions, the higher the reaction temperature is, the more is the reaction rate and the less is the time required until equilibrium is reached. However, the decomposition rate, too, rises. Therefore, the optimum reaction temperature is preferably controlled to fall within a range of from higher than room temperature to less than 60°C. The current density is preferably 0.1-100 $A/dm^2$. The distance between the electrodes is preferably as small as possible to reduce the loss due to resistance. However, the distance between the electrodes is preferably 1-50 mm to reduce the pressure loss of the pump during the supply of water and hence maintain the pressure distribution.

The concentration of hydrogen peroxide and peracetic acid thus produced can be controlled to 50,000 ppm (5% by weight) by adjusting the amount of water and the current density. The adjustment of concentration may be carried out also by combining an anode and a cathode having different catalysts.

In order to enhance the reaction efficiency, the electrolyte is preferably controlled and maintained acidic, i.e., to a pH range of 2-6. When the electrolyte is acidic, even if tap water is used as raw water, it is advantageous in that Ca ion and Mg ion contained in tap water are not deposited on the cathode in the form of hydroxide or carbonate.

By supplying raw water at a reduced rate and prolonging the contact time, the reaction for synthesis of peracetic acid is theoretically allowed to proceed until equilibrium is reached. However, synthesis is preferably conducted continuously for a practical period of time. Otherwise, the reaction product has much starting materials left behind therein. In the invention, as a starting material there is used acetic acid or acetate. The higher the concentration of the starting material is, the higher is the rate of production of peracetic acid. However, taking into account the safety in handling peracetic acid or hydrogen peroxide thus produced, the concentration of peracetic acid and hydrogen peroxide in the aqueous solution of starting material each are preferably 5% or less. The concentration of acetic acid as starting material, peracetic acid and hydrogen peroxide in the circulating sterilizing/cleaning water preferably fall within a range of 100-10,000 ppm, 10-4,000 ppm and 10-20,000 ppm, respectively.

As the material of electrolytic cell there is preferably used glass-lined material, carbon, titanium or stainless steel, which exhibits an excellent corrosion resistance, PTFE resin or the like.

Examples of the starting material employable herein include free acetic acid, and acetate such as sodium acetate and potassium acetate. The object to be cleaned by the sterilizing/cleaning method of the invention is not specifically limited but may be a vessel for cold drinks or beer or a medical tool such as endoscope.

An embodiment of sterilizing/cleaning with peroxide according to the method of the present invention will be described in connection with the attached drawings, but the invention is not limited thereto.

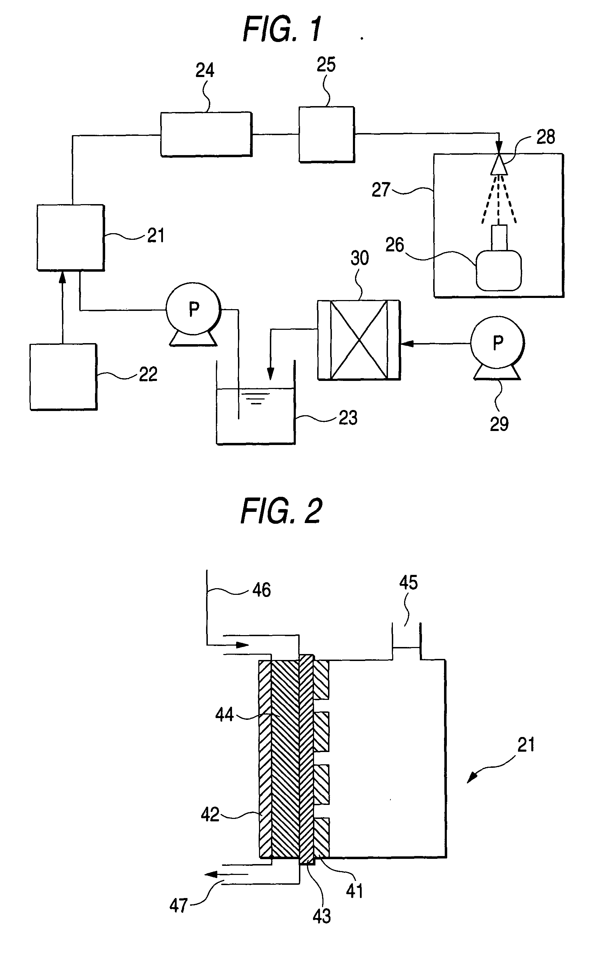

Fig. 1 is a schematic diagram illustrating an embodiment of the sterilizing/cleaning method with peracetic acid according to the present invention, and Fig. 2 is a schematic longitudinal sectional view of the electrolytic cell used in Fig. 1.

In the present embodiment, peracetic acid is synthesized in an electrolytic cell while an aqueous solution of peroxide which has been used for sterilizing/cleaning of an object to be cleaned is being circulated through the electrolytic cell for reuse.

An electrolytic cell 21 in Fig. 2 comprises an anode 41 made of expanded mesh or the like, a sheet-like oxygen gas cathode 42 and a membrane 43 which separates an anode chamber and a cathode chamber from each other as shown in Fig. 2. A catholyte defined by the oxygen gas cathode 42 and the membrane 41 is filled with a particulate solid acid catalyst such as Nafion resin or a fibrous catalyst material. The distance between the electrodes is kept to a range of from 1 to 50 mm. In Fig. 2, the reference numerals 45, 46 and 47 indicate an anode gas inlet, a catholyte inlet and a catholyte outlet, respectively.

Into the electrolytic cell 21 are supplied an oxygen gas from an oxygen supplying device 22 for preparing high purity oxygen in PSA process and a mixture of peracetic acid and hydrogen peroxide replenished with acetic acid from a storage tank 23 to produce peracetic acid and hydrogen peroxide from the acetic acid therein. The sterilizing/cleaning water containing peracetic acid and hydrogen peroxide (aqueous solution of peroxide) is detected for concentration of peracetic acid and hydrogen peroxide by a concentration sensor 24, and then heated by a heating device 25.

The sterilizing/cleaning water which has been detected for concentration is supplied into a sterilizing/cleaning chamber 27 receiving an object 26 to be sterilized/cleaned where it is then sprayed onto the object 26 through a nozzle 28. The sterilizing/cleaning water which has thus been used for sterilizing/cleaning is introduced into a decomposition product removing filter 30 from the sterilizing/cleaning chamber 27 by a first circulating pump 29. The dead body of living things and other impurities are removed through this filter. Thereafter, the sterilizing/cleaning water is stored in the storage tank 23 wherein it is then replenished with acetic acid in an amount consumed as necessary. The sterilizing/cleaning water is then circulated to the electrolytic cell by a second circulating pump 31 for reuse.

In accordance with the present embodiment, the sterilizing/cleaning water containing peracetic acid which has heretofore been discarded is circulated through the electrolytic cell for reuse, making it possible to prevent the deterioration of living environment due to discharge and synthesize peracetic acid from acetic acid or acetate, which is inexpensive. Further, since the necessity of the reduction of TOC value, etc., is eliminated, economic sterilizing/cleaning can be made.

Examples of the sterilizing/cleaning method with peroxide according to the invention will be described hereinafter, but the invention is not limited thereto.

**EXAMPLE 1**

Iridium oxide was deposited on a porous titanium sheet in a proportion of 10 g/m$^2$ by thermal decomposition method to form an anode. A graphite powder (TGP-2, produced by Tokai Carbon Co., Ltd.) was then kneaded with a PTFE resin as a catalyst. The kneaded mixture was spread over a carbon cloth (PWB-3, produced by Zoltek Corporation) as a core material, and then calcined at 330°C to prepare a 0.5 mm thick sheet as an oxygen gas cathode. As a membrane there was used an ion-exchange membrane Nafion (trade name) 117 (produced by DuPont Inc.). The distance between the membrane and the oxygen gas cathode was 5 mm. The space between the membrane and the oxygen was filled with Nafion resin (NR-50, produced by DuPont Inc.). The distance between the ion-exchange membrane and the anode was 0 mm. Thus, an electrolytic cell having an effective electrolysis area was formed.

At a temperature of 25°C, an electric current of 10A was passed through the electrolytic cell while air being supplied into the gas chamber at a rate of 200 ml per minute and an aqueous solution of acetic acid (5M $CH_3COOH$, pH 3) being each supplied into the anode chamber and the cathode chamber of the cell at a rate of 10 ml per minute. The resulting cell voltage was 8 V. The solution from the cathode outlet was then measured for concentration of hydrogen peroxide by titration with $KMnO_4$ and concentration of peracetic acid using an HPLC liquid chromatograph. As a result, it was found that 240 ppm of peracetic acid and 1,200 ppm of hydrogen peroxide had been obtained.

**EXAMPLE 2**

The electrolysis procedure of Example 1 was followed except that a membrane-free electrolytic cell having no ion-exchange membrane as a membrane was used. The resulting cell voltage was 8 V. 220 ppm of peracetic acid and 100 ppm of hydrogen peroxide were obtained at the cathode outlet.

**EXAMPLE 3**

The electrolysis procedure of Example 1 was followed except that as the catalyst for oxygen gas cathode there was used a graphite powder having a platinum content of 0.5 mg/cm$^2$. The resulting cell voltage was 7.5 V. The solution from the cathode outlet was then measured for concentration of hydrogen peroxide by titration with $KMnO_4$ and concentration of peracetic acid using an HPLC liquid chromatograph. As a result, it was found that 600 ppm of hydrogen peroxide and 800 ppm of peracetic acid had been obtained.

**EXAMPLE 4**

The electrolysis procedure of Example 1 was followed except that an electric current of 20 A was passed through the electrolytic cell and air was supplied into the gas chamber at a rate of 500 ml per minute. The resulting cell voltage was 7.5 V. 300 ppm of hydrogen peroxide and 800 ppm of peracetic acid were obtained at the cathode outlet. The comparison of Examples 1 and 4 shows that the ratio of concentration of hydrogen peroxide and peracetic acid can be adjusted by current density.

**EXAMPLE 5**

The electrolytic cell of Example 1 was assembled into a circulating line as shown in Fig. 1 (No concentration sensor was provided). The aqueous solution of peroxide containing hydrogen peroxide and peracetic acid in the concentration as obtained in Example 1 was brought into contact with an object to be cleaned in the sterilizing/cleaning chamber for 1 minute, and then recovered. The aqueous solution of peroxide thus recovered contained dead bacteria in a concentration of about 1,000 pieces/ml. When the aqueous solution was filtered through a UF filter, the number of bacteria was zero.

**EXAMPLE 6**

The solution from the cathode outlet obtained in Example 1 was diluted by a factor of two to half the concentration of peracetic acid and hydrogen peroxide (120 ppm of peracetic acid and 600 ppm of hydrogen peroxide). The diluted solution was then supplied into the electrolytic cell of Example 1 for electrolysis. As a result, the concentration of peracetic acid and hydrogen peroxide in the solution from the cathode outlet were about 240 ppm and 1,200 ppm, respectively, which are equal to the value measured before circulation. This demonstrates that the aqueous solution of peroxide can be circulated.

**EXAMPLE 7**

The electrolytic cell of Example 1 was assembled into a circulating line comprising a concentration sensor as shown in Fig. 1. The circulating line was then continuously operated for 1,000 hours while the current passing through the electrolytic cell was being varied on the basis of the concentration of peracetic acid detected by the concentration sensor and the concentration of peracetic acid at the concentration sensor was being kept almost constant. As a result, the concentration of peracetic acid in the sterilizing/cleaning water to be fed to the sterilizing/cleaning chamber was kept at a range of 250-300 ppm.

The present invention concerns a method for the sterilizing/cleaning of an object with an aqueous solution of a peroxide, which comprises sterilizing/cleaning the object with an aqueous solution containing a peracetic acid electrolytically synthesized from acetic acid and/or acetate and an oxygen-containing gas as starting materials.

In accordance with the method of the present invention, peracetic acid can be synthesized from acetic acid or acetate, which is inexpensive. Drink containers, etc. can be efficiently sterilized and cleaned with a sterilizing/cleaning water containing the peracetic acid thus synthesized.

In accordance with the present invention, the sterilizing/cleaning water used for sterilizing/cleaning is circulated through the electrolytic cell. In other words, the sterilizing/cleaning water containing peracetic acid which has heretofore been discarded is circulated through the electrolytic cell for reuse, making it possible to prevent the deterioration of living environment due to discharge. Further, since the necessity of the reduction of TOC value, etc. is eliminated, economic sterilizing/cleaning can be made.

In accordance with the apparatus of the present invention comprising a filter for removing impurities such as dead body of living things, the purity of the aqueous solution of peroxide to be circulated through the electrolytic cell can be further enhanced, making it possible to raise the electrolysis efficiency and prevent troubles with electrolytic cell due to impurities.

When a concentration sensor is provided in the circulating line, the concentration of peracetic acid can be kept within a predetermined range by adjusting the amount of electricity to be supplied into the electrolytic cell depending on the magnitude of the concentration of peracetic acid continuously measured by the concentration sensor, making it possible to exert a desired sterilizing/cleaning effect.

Further, the method of the present invention allows easy electrolytic synthesis of peracetic acid from acetic acid or acetate, which is inexpensive, and thus is an epochal method for the production of peracetic acid.

It should further be apparent to those skilled in the art that various changes in form and detail of the invention as shown and described above may be made. It is intended that such changes be included within the spirit and scope of the claims appended hereto.

This application is based on Japanese Patent Application No. 2003-095501 filed March 31, 2003, the disclosure of which is incorporated herein by reference in its entirety.

**Claims**

1. A method for the sterilizing/cleaning of an object with an aqueous solution of a peroxide, which comprises sterilizing/ cleaning the object with an aqueous solution containing a peracetic acid electrolytically synthesized from acetic acid and/or acetate and an oxygen-containing gas as starting materials.

2. The method as claimed in Claim 1, wherein the aqueous solution of a peroxide used for the sterilizing/cleaning of the object is reused for electrolytic synthesis.

3. A sterilizing/cleaning apparatus using a peroxide comprising an electrolytic cell for performing electrolysis while being supplied with acetic acid and/or acetate and an oxygen-containing gas to synthesize an aqueous solution containing peracetic acid and hydrogen peroxide at the cathode, a sterilizing/cleaning chamber for allowing the aqueous solution produced in the electrolytic cell to come in contact with an object, a filter disposed downstream the chamber for filtering the aqueous solution of a peroxide containing peracetic acid and hydrogen peroxide from the sterilizing/cleaning chamber to remove the dead body of living things and a unit for circulating the aqueous solution of peroxide filtered through the filter to the electrolytic cell.

4. The sterilizing/cleaning apparatus as claimed in Claim 3, wherein a sensor for measuring the concentration of peracetic acid in the aqueous solution of peroxide is provided in the circulation line.

5. A method for the electrolytic synthesis of peracetic acid which comprises electrolytically synthesizing peracetic acid from acetic acid and/or acetate and an oxygen-containing gas as starting materials in the presence of a solid acid catalyst.

# FIG. 1

# FIG. 2

## FIG. 3

**EP 1 469 102 A1**

**European Patent Office**

**EUROPEAN SEARCH REPORT**

Application Number

EP 04 00 7700

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01/10215 A (STERIS INC.) 15 February 2001 (2001-02-15) | 1-4 | C25B1/28 A61L2/03 |
| Y | * page 15, line 3 - line 28 * * page 23, line 2 - line 27 * * page 26, line 2 - page 27, line 13 * * page 44; claims 1,4 * * figures 1,3 * | 5 | |
| Y | WO 02/068567 A (SOLVAY) 6 September 2002 (2002-09-06) * page 2, line 17 - line 29 * | 5 | |
| P,X | PATENT ABSTRACTS OF JAPAN vol. 2003, no. 12, 5 December 2003 (2003-12-05) & JP 2004 043365 A (PERMELEC ELECTRODE LTD; RIKOGAKU SHINKOKAI), 12 February 2004 (2004-02-12) * abstract * | 1,5 | |
| A | US 6 171 551 B1 (PAUL S. MALCHESKY) 9 January 2001 (2001-01-09) * column 11; example 6 * | 1-4 | TECHNICAL FIELDS SEARCHED (Int.Cl.7) C25B A61L |

The present search report has been drawn up for all claims

| Place of search | Date of completion of the search | Examiner |
|---|---|---|
| The Hague | 25 August 2004 | Groseiller, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

12

ANNEX TO THE EUROPEAN SEARCH REPORT
ON EUROPEAN PATENT APPLICATION NO.          EP 04 00 7700

This annex lists the patent family members relating to the patent documents cited in the above–mentioned European search report.
The members are as contained in the European Patent Office EDP file on
The European Patent Office is in no way liable for these particulars which are merely given for the purpose of information.

25–08–2004

| Patent document cited in search report | | Publication date | Patent family member(s) | | Publication date |
|---|---|---|---|---|---|
| WO 0110215 | A | 15–02–2001 | AT | 245351 T | 15–08–2003 |
| | | | AU | 764557 B2 | 21–08–2003 |
| | | | AU | 6757900 A | 05–03–2001 |
| | | | BR | 0013008 A | 03–12–2002 |
| | | | CA | 2378601 A1 | 15–02–2001 |
| | | | CN | 1377229 T | 30–10–2002 |
| | | | DE | 60004060 D1 | 28–08–2003 |
| | | | DE | 60004060 T2 | 22–04–2004 |
| | | | EP | 1199931 A1 | 02–05–2002 |
| | | | ES | 2203505 T3 | 16–04–2004 |
| | | | JP | 2003506120 T | 18–02–2003 |
| | | | NZ | 517172 A | 26–09–2003 |
| | | | WO | 0110215 A1 | 15–02–2001 |
| | | | US | 6387238 B1 | 14–05–2002 |
| WO 02068567 | A | 06–09–2002 | FR | 2821350 A1 | 30–08–2002 |
| | | | CA | 2439162 A1 | 06–09–2002 |
| | | | WO | 02068567 A1 | 06–09–2002 |
| | | | EP | 1377652 A1 | 07–01–2004 |
| | | | US | 2004154959 A1 | 12–08–2004 |
| JP 2004043365 | A | 12–02–2004 | NONE | | |
| US 6171551 | B1 | 09–01–2001 | AT | 238074 T | 15–05–2003 |
| | | | AU | 742315 B2 | 20–12–2001 |
| | | | AU | 2489499 A | 23–08–1999 |
| | | | BR | 9907675 A | 05–03–2002 |
| | | | CA | 2320482 A1 | 12–08–1999 |
| | | | CN | 1292710 T | 25–04–2001 |
| | | | DE | 69907143 D1 | 28–05–2003 |
| | | | DE | 69907143 T2 | 25–03–2004 |
| | | | EP | 1051200 A1 | 15–11–2000 |
| | | | ES | 2198891 T3 | 01–02–2004 |
| | | | JP | 2002502636 T | 29–01–2002 |
| | | | WO | 9939753 A1 | 12–08–1999 |
| | | | RU | 2207881 C2 | 10–07–2003 |

EPO FORM P0459

For more details about this annex : see Official Journal of the European Patent Office, No. 12/82